# EUROPEAN PATENT APPLICATION

(11) **EP 4 289 930 A1**
(43) Date of publication of application: **13.12.2023**
(21) Application number: 22745071.5
(22) Date of filing: 17.01.2022
(51) Int. Cl.: C12M 3/00

(54) **THAW RECOVERY SYSTEM AND METHOD FOR BIOMATERIAL**

(30) Priority: 01.02.2021 CN 202110136270
(71) Applicant: Shen Zhen Biorocks Biotechnology Company Limited, Shenzhen, Guangdong 518000 (CN)
(72) Inventor: CHAN, Ho Nam, Hong Kong (HK); LEE, Tak Sum, Hong Kong (HK)
(74) Representative: Clarke, Modet y Cía., S.L.
(86) International application number: PCT/CN2022/072245
(87) International publication number: WO 2022/161195

(57) **Abstract**

Disclosed in the present invention is a system of thawing biological materials and method thereof, wherein the system comprises: a carrier, a container and a chip. a recess is located near a front end of the carrier, in which a biological material to be thawed and a cryoprotectant are loaded; an oil-phase thawing fluid is loaded in the container, immersing the recess of the carrier into the oil-phase thawing fluid, the oil-phase thawing fluid covers the recess of the carrier and thaws and rewarms the biological material; a chemical including a cryoprotectant and/or a basic solution is encapsulated in the chip, removing the carrier from the oil-phase thawing fluid and covering the carrier with the chip; moving the chip relative to the carrier, the chemical contacts the recess in a full-covering manner for removing the cryoprotectant in the recess. The process of the entire thawing process could be effectively standardized and automated through the present application, which realizes effective quality control, increases processing efficiency, and reduces personnel training requirements.

## Description

### Field of the Invention

The invention relates to bioengineering field, particularly to a system of thawing biological materials and method thereof.

### Related Art

Cell cryopreservation technologies are significant in many biological fields, meanwhile, recovering the cells from a low-temperature frozen state form to a normal metabolic state is also important. This thawing process includes: removing the cryoprotectants, and replacing them by normal culture media.

In the prior art, the demand for the thawing process is relatively high, and this process is cumbersome, difficult, highly focused, stressful and difficult to standardize.

### SUMMARY OF THE INVENTION

The main aspect of the present disclosure provides a system of thawing biological materials and the method thereof, for solving the technical issue related to the low efficiency of the manual thawing process in the art.

According to the embodiments of the present disclosure, a system of thawing biological materials is proposed, the system comprises: a carrier with a recess located near a front end of the carrier, in which a biological material to be thawed and a cryoprotectant are loaded; a container, for loading an oil-phase thawing fluid used to cover the recess of the carrier immersed therein and to thaw and rewarm the biological material; a chip encapsulating a chemical including a cryoprotectant and/or a basic solution; with the chip moving relative to the carrier, the chemical contacts the recess in a full-covering manner for removing the cryoprotectant in the recess.

The specific embodiments may include one or more of the following technologies. The cryoprotectant contained in the chemical is a non-permeable cryoprotectant. The chemical includes a thawing solution, a dilution solution and a washing solution, which are made in form of hydrogels; the chip is provided with a plurality of sections used for embedding a thawing solution, a dilution and a washing solution in sequence, wherein coverage area of each section is larger than opening area of the recess; while the chip moves relative to the carrier, the thawing solution, the dilution solution and the washing solution are capable of move to upper side of the recess sequentially and contact the recess in a full-covering manner sequentially. The chemical includes a thawing solution, a dilution solution, and a washing solution, which are made in form of solutions; the chip is provided with a permeable membrane for supporting the thawing solution, the dilution solution and the washing solution; wherein the permeable membrane is one of a perforated membrane, a mesh, a dialysis membrane or a water-soluble membrane. Prior to the chip moving relative to the carrier, the chemical covers a part area of the carrier excluding the recess of the carrier, and an interstice distance is provided between the chemical and the recess of the carrier. A substrate, a middle area of the substrate is used to place the carrier, and two rails paralleling with each other for supporting and fixing the chip are provided along a length direction of the substrate, to keep contacting between a lower surface of the chip and an upper surface of the carrier.

According to the embodiments of the present disclosure, a system of thawing biological materials, is further proposed, the system includes: a first carrier loading a biological material to be thawed and a cryoprotectant; a container loading a thawing solution used to cover the first carrier immersed therein and to thaw and rewarm the biological material to separate the biological material from the first carrier; a second carrier with a recess located near a front end of the second carrier, in which a first dilution solution is loaded; a capillary tube used to transfer the biological material into the recess of the second carrier; a chip encapsulating a chemical including a cryoprotectant and/or a basic solution; with the chip moving relative to the second carrier, the chemical contacts the recess of the second carrier in a full-covering manner, for removing the cryoprotectant in the recess.

The specific embodiments may include one or more of the following technologies. The cryoprotectant contained in the chemical is a non-permeable cryoprotectant. The chemical includes a second dilution solution and washing solution made in form of hydrogels, the second dilution solution contains a non-permeable cryoprotectant with a concentration lower than the concentration of the non-permeable cryoprotectant in the first dilution solution; the chip is provided with a plurality of sections for embedding a second dilution hydrogel and a washing hydrogel in sequence, wherein coverage area of each section is larger than opening area of the recess; while the chip moves relative to the carrier, the second dilution hydrogel and the washing hydrogel move sequentially to an upper side of the recess and contact the recess in a full-covering manner sequentially. The chemical includes a second dilution solution and a washing solution, which are made in form of solutions; wherein a concentration of the non-permeable cryoprotectant in the second dilution solution is lower than a concentration of the non-permeable cryoprotectant in the first dilution solution; the chip is provided with a permeable membrane, and the permeable membrane is used to support the second dilution solution and the washing solution in solution form; wherein the permeable membrane is one of a perforated membrane, a mesh, a dialysis membrane or a water-soluble membrane. Prior to the chip moving relative to the second carrier, the chemical covers a part area of the second carrier excluding the recess of the second carrier, and an interstice distance is provided between the chemical and the recess of the second carrier. A substrate, a middle area of the substrate is used to place the second carrier, and two rails paralleling with each other for supporting and fixing the chip are provided along the length direction of the substrate, to keep contacting between a lower surface of the chip and an upper surface of the second carrier.

According to the embodiments of the present disclosure, a method of thawing biological materials, is further proposed. The method comprises: providing a carrier with a recess near the front end thereof, in which a biological material to be thawed and a cryoprotectant are located; immersing the recess of the carrier in an oil-phase thawing fluid, the oil-phase thawing fluid is used to cover the recess of the carrier and to thaw and rewarm the biological material; providing a chip encapsulating a chemical including a cryoprotectant and/or a basic solution; removing the carrier from the oil-phase thawing fluid, and covering the carrier with the chip; moving the chip relative to the carrier, the chemical contacts the recess in a full-covering manner, for removing the cryoprotectant in the recess.

The specific embodiments may include one or more of the following technologies. The cryoprotectant contained in the chemical is a non-permeable cryoprotectant. Prior to the chip moving relative to the carrier, the chemical covers a part area of the carrier excluding the recess of the carrier, and an interstice distance is provided between the chemical and the recess of the carrier. The chemical includes a thawing solution, a dilution solution and a washing solution made in form of hydrogels; the chip is provided with a plurality of sections for embedding a thawing hydrogel, a dilution hydrogel and a washing hydrogel in sequence, wherein coverage area of each section is larger than opening area of the recess; while the chip moves relative to the carrier, the thawing solution, the dilution solution and the washing solution move sequentially to an upper side of the recess and contact the recess in a full-covering manner sequentially. The chemical includes a thawing solution, a dilution solution, and a washing solution made in form of solutions; the chip is provided with a permeable membrane for supporting the thawing solution, the dilution solution and the washing solution; wherein the permeable membrane is one of a perforated membrane, a mesh, a dialysis membrane or a water-soluble membrane.

According to the embodiments of the present disclosure, a method of thawing biological materials, is further proposed. The method comprises: providing a first carrier loading a biological material to be thawed and a cryoprotectant; immersing the first carrier into a thawing solution, the thawing solution is used to cover the first carrier and to thaw and rewarm the biological material; providing a second carrier with a recess near the front end thereof, a first dilution solution is loaded in the recess of the second carrier; transferring the biological material into the recess of the second carrier; providing a chip encapsulating a chemical and covering the second carrier with the chip; wherein the chemical includes a cryoprotectant and/or a basic solution; moving the chip relative to the second carrier, the chemical contacts the recess of the second carrier in a full-covering manner, for removing the cryoprotectant in the recess.

The specific embodiments may include one or more of the following technologies. The cryoprotectant contained in the chemical is a non-permeable cryoprotectant. Prior to the chip moving relative to the carrier, the chemical covers a part area of the second carrier excluding the recess of the second carrier, and an interstice distance is provided between the chemical and the recess of the second carrier. The chemical includes a second dilution solution and a washing solution made in form of hydrogels, the second dilution solution contains a non-permeable cryoprotectant with a concentration lower than a concentration of the non-permeable cryoprotectant contained in the first dilution solution; the chip are provided a plurality of sections for embedding a second dilution hydrogel and a washing hydrogel in sequence, wherein coverage area of each section is larger than opening area of the recess; while the chip moves relative to the carrier, the second hydrogel solution and the washing hydrogel move sequentially to an upper side of the recess and contact the recess in a full-covering manner sequentially. The chemical includes a second dilution solution and a washing solution made in form of solutions; wherein the concentration of the non-permeable cryoprotectant in the second dilution solution is lower than the concentration of the non-permeable cryoprotectant in the first dilution solution; the chip is provided with a permeable membrane to support the second dilution solution and the washing solution; wherein the permeable membrane is one of a perforated membrane, a mesh, a dialysis membrane or a water-soluble membrane.

According to the embodiments of the present disclosure, a method of thawing biological material, is further proposed. The method comprises: preparing for a chemical in hydrogel form, wherein the chemical contains a cryoprotectant and/or a basic solution; making the biological material including the cryoprotectant contact with the hydrogel, thereby the cryoprotectant is diffused and removed by spreading the cryoprotectant to the hydrogel.

The specific embodiments may include one or more of the following technologies. The cryoprotectant contained in the chemical is a non-permeable cryoprotectant. The step of preparing for the chemical into a form of hydrogel comprises dissolving an agarose into a chemical solution at 80-90°C for preparing for a 0.1-6% agarose solution; obtaining an agarose gel containing the chemical by stirring, mixing, cooling, and solidifying. The step of preparing the chemical into a hydrogel structure comprises dissolving sodium alginate into the chemical solution for preparing for a 0.3-10% sodium alginate solution; dissolving calcium chloride in the chemical solution for preparing a 0.01-0.2M calcium chloride solution; providing the calcium chloride solution onto the sodium alginate solution in the chip; when the calcium chloride is diffused to the bottom of the chip, solidifying the whole piece of sodium alginate solution into calcium alginate hydrogel. The step of preparing for the chemical into a hydrogel structure comprises dissolving gelatin into a chemical solution at 36-45°C to make a 1-15% gelatin solution; applying the gelatin solution at 36-50°C into the chip; cooling and solidifying the gelatin solution for configuring a hydrogel with the chemical. The step of preparing the chemical into a hydrogel structure comprises dissolving GelMA into the chemical solution at 36-50°C for preparing a 1-15% GelMA solution; dissolving a photo initiator into the GelMA solution at 36-50°C with a concentration of 0.001-2%; applying the GelMA solution at 36-50°C to the chip; irradiating the GelMA solution at 36-50°C for 5-30 minutes for solidifying the GelMA solution into a hydrogel. The chemicals include one or more of a thawing solution, a dilution solution or a washing solution.

According to the technologies in the present disclosure, after the biological materials are thawed and rewarmed, the carrier with embryos is covered by a chip encapsulating a chemical, the chemical is capable of contacting the recess in a full-covering manner, for removing the cryoprotectant in the recess, thus the process of the entire thawing process could be effectively standardized and automated, for realizing effective quality control, increasing processing efficiency, and reducing personnel training requirements.

### BRIEF DESCRIPTION OF THE DRAWINGS

The drawings described here are used to provide a further understanding of the present disclosure and constitute a part of the present disclosure, the exemplary embodiments and the description thereof are used to illustrate the present disclosure, but not used to limit the present application. In the drawings:
Fig. 1 is a flowchart of a method of thawing biological materials according to an embodiment of the present disclosure;
Fig. 2 is a flowchart of a method of thawing biological materials according to another embodiment of the present disclosure;
Fig. 3 is a flowchart of a method of thawing biological materials according to another embodiment of the present disclosure;
Fig. 4 is a flowchart of a method of thawing biological materials according to another embodiment of the present disclosure;
Fig. 5 is a schematic drawing of a carrier and a container according to an embodiment of the present disclosure;
Fig. 6 is a schematic drawing of a carrier according to an embodiment of the present disclosure;
Fig. 7 is a schematic drawing of a chip according to an embodiment of the present disclosure;
Fig. 8 is a schematic drawing of a biological material thawing system according to an embodiment of the present disclosure;
FIG. 9 is a schematic drawing of the structure of a substrate according to an embodiment of the present disclosure;
Fig. 10 is a schematic drawing of a biological material thawing system according to another embodiment of the present disclosure;
Fig. 11 is a schematic drawing of a biological material thawing system according to still another embodiment of the present disclosure.

### DETAILED DESCRIPTION OF THE INVENTION

For clarifying the objectives, technologies, and advantages of the present disclosure, the technologies proposed in the present disclosure will be described clearly and completely in conjunction with specific embodiments and the corresponding drawings thereof in the present disclosure. Obviously, the embodiments described herein are merely a part of the embodiments of the present disclosure, rather than all of them. Based on the embodiments according to the present disclosure, all other embodiments obtained by those of ordinary skill in the art without creative work should fall within the protective scope required by the present disclosure.

With reference to the accompanying drawings, the technologies according to the embodiments in the present disclosure will be described in detail as follow.

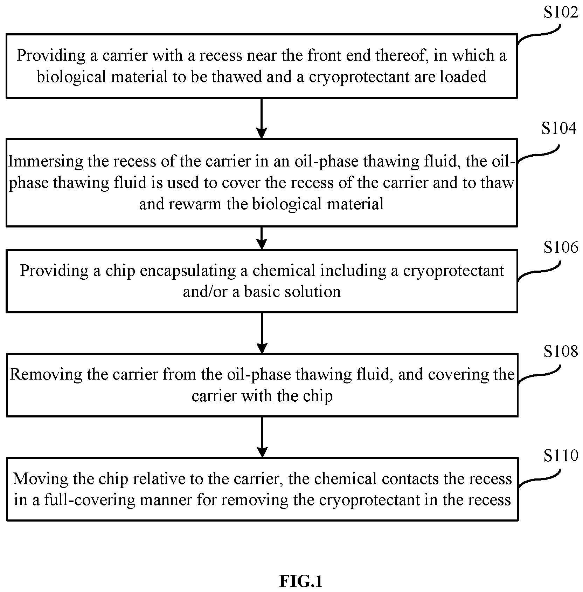

Refer to Fig. 1, one embodiment of the present application proposes a thawing method of biological materials, specifically, this method includes the following steps:
Step S 102, providing a carrier with a recess near the front end thereof, in which a biological material to be thawed and a cryoprotectant are loaded.

The carrier with the biological material is stored in a low-temperature liquid nitrogen environment (e.g., -196°C), and the cryoprotectant used to protect the biological material is disposed near or around the biological material. Wherein, the biological material could be cells, biological tissues, eggs or embryos, and the cryoprotectant could be vitrification solution ("VS" for short) used to protect the biological material.

Step S104, immersing the recess of the carrier in an oil-phase thawing fluid, the oil-phase thawing fluid is used to cover the recess of the carrier and to thaw and rewarm the biological material. Wherein, the oil-phase thawing fluid does not contain any non-permeable cryoprotectant.

The carrier is capable of being quickly moved from a low temperature environment to a container loading an oil-phase thawing fluid, wherein the recess of the carrier should be immersed in the oil-phase thawing fluid, so as to ensure the heating rate. Since the density of the oil-phase thawing fluid is lower than the density of the cryoprotectant, the cryoprotectant along with the embryo is capable of being left in the recess, rather than drifts out of the recess. This step is capable of being used to quickly warm and thaw the embryo/egg in the recess.

Step S106, providing a chip encapsulating a chemical including a cryoprotectant and/or a basic solution.

According to the present application, the concentration of the cryoprotectant included in the chemical is less than the concentration of the cryoprotectant in the recess. In practice, the cryoprotectant included in the chemical could be a non-permeable cryoprotectant or a permeable cryoprotectant. Specifically, when the cryoprotectant in the embryos is removed by the chemical, the chemical may include cryoprotectant and/or basic solution, alternatively, the chemical may include non-permeable cryoprotectant and/or basic solution. For obtaining a better removal effect, the chemical including non-permeable cryoprotectant and/or basic solution could be used. The main reason lies in that the non-permeable cryoprotectant cannot pass through the cell membrane and are generally non-cytotoxic molecules. Meanwhile, the permeable cryoprotectant generally has small molecules with cytotoxicity. In the thawing application, the function of the non-permeable cryoprotectant is ensuring that the osmotic pressure difference between the inner side of cell and outside of the cell is not too much. In other words, in the thawing application, the inner of the cells is filled with the permeable cryoprotectant when the cells are taken out from the liquid nitrogen, thus the osmotic pressure inside the cells is very high, and if these cells are placed into a media directly during the thawing process, the water molecules are capable of entering the cells very quickly and killing these cells under this osmotic pressure difference. However, if the cells are placed into the solution including the non-permeable cryoprotectant during the thawing process, the speed of water molecules entering the cell could be reduced and the permeable cryoprotectant could diffuse out of the cell due to a lower osmotic pressure difference, thereby the purpose of removing the cryoprotectant slowly could be accomplished.

Wherein, the chemical include: thawing solution, dilution solution and washing solution. In the embodiments according to the present disclosure, the chemicals may in solid made of hydrogel or in solution.

As for the chemical in solid, the thawing solution, dilution solution and washing solution are in hydrogel form, namely, thawing hydrogel (TS gel), dilution hydrogel (DS gel) and washing hydrogel (WS gel), are embedded into the chip. Wherein, the hydrogel is a kind of solid material of which the main component is liquid, meanwhile, the hydrogel is lacking of fluidity due to the three-dimensional cross-linked network in the gel. Hydrogels could be either very soft or very hard. The liquid in the gel includes a non-permeable cryoprotectant and/or basic solution. Specifically, the thawing hydrogel includes a basic solution and a non-permeable cryoprotectant having extremely high concentration, e.g., the non-permeable cryoprotectant with a concentration of 0.5M-1.5M. The DS gel includes a basic solution and a non-permeable cryoprotectant with a concentration lower than the concentration of the TS gel, e.g., the non-permeable cryoprotectant with a concentration of 0.1M-0.75M. The WS gel only includes the basic solution.

Specifically, the chip is capable of being provided with three sections arranged thereon in sequence, and thus the TS gel, the DS gel (i.e. a first DS gel), and the WS gel are capable of being embedded into these sections in sequence, wherein the coverage area of each section is larger than the opening area of the recess in the carrier. Moreover, the chip is capable of being provided with four sections arranged thereon in sequence, the extra section is used to embed a second DS gel, wherein the concentration of the non-permeable cryoprotectant in the second DS gel is lower than the concentration of the non-permeable cryoprotectant in the first DS gel, thus the damage on the embryos caused by the osmotic pressure could be released, as the osmotic pressure changes relatively slowly.

As for the chemical in solution, the thawing solution, the dilution solution and the washing solution in solution are encapsulated within the chip by a permeable membrane. The concentrations of the basic solution and the non-permeable cryoprotectant included in the chemical in solution are similar to those of the hydrogel and will not be repeated here.

Specifically, the chip is capable of being provided with three sections arranged thereon in sequence, and thus the thawing solution, dilution solution and washing solution could be encapsulated into these sections in sequence, wherein the coverage area of each section is larger than the opening area of the recess. Moreover, the chip could be provided with four sections sequentially arranged thereon, wherein the extra section is used to encapsulate a second dilution solution, wherein the concentration of the non-permeable cryoprotectant contained in this second dilution solution is lower than the concentration of the non-permeable cryoprotectant contained in this first dilution solution, thus the damage on the embryos caused by the osmotic pressure will be released as the osmotic pressure changes relatively slowly. In practice, a perforated membrane, mesh or dialysis membrane with suitable thickness and suitable pore size could be chosen to be provided on the lower surface of the chip, meanwhile, the solutions could be supplied into the sections directly, thereby, the solutions are capable of being supported by the perforated membrane, the mesh, the dialysis membrane, or the water-soluble membrane, and thus the solutions are capable of being immobilized in the chip.

Step S 108, removing the carrier from the oil-phase thawing fluid, and covering the carrier with the chip.

After the re-warming process is completed (generally, merely by being immersing in the oil-phase thawing fluid for 2-3 seconds), the chip which encapsulates the chemical is arranged on the carrier. In order to obtain a better diffusion removal effect, the thawing solution, the dilution solution and the washing solution in hydrogel form or in solution form cover some parts of the carrier, i.e., do not cover the recesses of the carrier, and keep a distance of several millimeters from the recesses of the carrier.

Step S 110, moving the chip relative to the carrier, the chemical contacts the recess in a full-covering manner for removing the cryoprotectant in the recess.

In the case of the chemical in solid hydrogel is immobilized in the chip, when the chip moves relative to the carrier, the thawing hydrogel, the dilution hydrogel and the washing hydrogel embedded in the chip are capable of being moved to the upper side of the recess of the carrier in sequence and are capable of sequentially contacting the biological material to be thawed in the recess in a full-covering manner. Since the molecules of the chemical are capable of freely diffusing in the hydrogel, the embryos and cryoprotectant in the recess are capable of diffusing into the hydrogel as the chemical contacts the recess on the carrier, thus function of removing the cryoprotectant is implemented. Specifically, as for the TS gel, the toxic permeable cryoprotectant with higher concentration in the embryo can be removed where the embryo is in a lower osmotic differential pressure state. As for the DS gel, the water is capable of re-diffusing into the embryo slowly, thus the molecules of the non-permeable cryoprotectant around the embryos are capable of being removed in sequence (if the removing process is performed quickly, the osmotic pressure might damage the embryos). As for the WS gel, because the more water is capable of re-diffusing into the embryo slowly, the molecules of the non-permeable cryoprotectant around the embryos are capable of being completely removed, thereby, the embryos are capable of being brought back to the environment having normal osmotic pressure. After being treated by the three kinds of hydrogels, there is not any cryoprotectant molecule in the embryos, meanwhile, the embryos are capable of being transferred from the carrier to the culture dish and are capable of being cultured in the incubator, in the environment which has been brought back to the environment having normal osmotic pressure.

In the case of the chemical in solution is immobilized in the chip, the embryos are prevented from drifting out by covering the recess with the permeable membrane, thus the diffusion-exchange of the solutions could be implemented on both sides of the permeable membrane when the solutions on the two sides of the permeable membrane contact to each other, please refer to the embodiment related to the hydrogel for details.

In general, no matter the chemical in solid or liquid is used, the embryos are capable of being blocked within the recess and the diffusion-exchange of the chemical in the recess is implemented, meanwhile, the chemical is prevented from drifting out of the chip. After the entire removal process is completed, the embryos are left in the recess of the carrier, thus the position of the embryos could be determined quickly for capturing the embryos, and then the embryos could be transferred from the carrier to the culture dish for culturing in the incubator. Since the medium composition of the washing hydrogel (or washing solution) is not necessarily be coincident with the user's medium composition, the user could put the embryos transferred from the carrier into the user's medium for performing the final washing according to his requirements at first, and then the embryos could be transferred to the user's culture system for culturing.

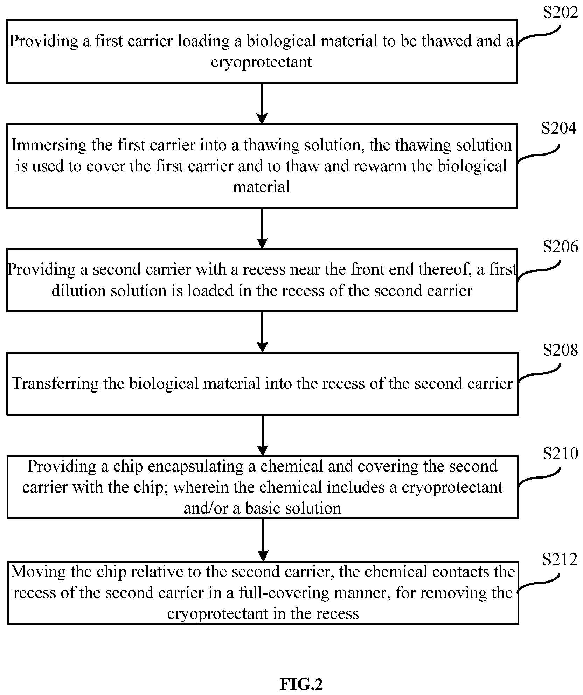

In the embodiment shown by Fig. 1, an oil-phase thawing fluid is used for thawing-rewarming process. In other embodiments, a non-oil-phase thawing fluid, i.e., a general thawing solution, can also be used for thawing- rewarming. Referring to FIG. 2, the method of thawing biological materials according to an embodiment of the present application includes the following steps:
Step S202, providing a first carrier loading a biological material to be thawed and a cryoprotectant.

The biological material carried by the first carrier is preserved in a low-temperature liquid nitrogen environment, the cryoprotectant used to protect the biological material is disposed near or around the biological material, wherein the biological material could be cells, biological tissues, eggs or embryos, wherein the cryoprotectant could be VS. For the sake of brevity, the embryo is taken as an example of the biological material in the following descriptions.

In some implementations, the front end of the first carrier could be provided with a recess, and the biological material is carried in the recess of the first carrier.

Step S204, immersing the first carrier into a thawing solution, the thawing solution is used to cover the first carrier and to thaw and rewarm the biological material.

The first carrier is quickly moved from the low temperature environment to the container containing the thawing solution, and then the embryos carried by the first carrier are completely immersed in the thawing solution, by this way, the thawing temperature rise rate could be ensured and the embryos could be separated from the first carrier.

Step S206, providing a second carrier with a recess near the front end thereof, a first dilution solution is loaded in the recess of the second carrier.

A second carrier having recesses provided at the front end thereof could be prepared in advance, and the recesses of the second carrier are filled with the first dilution solution, wherein the volume of the first dilution solution is required to be greater than the capacity of the recess.

Step S208, transferring the biological material into the recesses of the second carri er.

After the embryos are separated from the first carrier, the exact positions of the embryos could be determined, and then the embryos are transferred into the recess of the second carrier by a capillary tube(for example, by a glass capillary).

Step S210, providing a chip encapsulating a chemical and covering the second carrier with the chip, wherein the chemical includes a cryoprotectant and/or a basic solution.

In the embodiments of the present application, the concentration of the cryoprotectant contained in the chemical is less than the concentration of the cryoprotectant in the recess. In practice, the cryoprotectant contained in the chemical could be a non-permeable cryoprotectant or a permeable cryoprotectant. Specifically, when the cryoprotectant in embryos is removed by the chemical, the chemical may contain cryoprotectants and/or basic solution, alternatively, the chemical may contain non-permeable cryoprotectants and/or basic solution. For achieving a better removal effect, the chemical containing non-permeable cryoprotectants and/or basic solution could be used. The principle reason lies in that the non-permeable cryoprotectants cannot pass through cell membranes and are generally non-cytotoxic molecules. Generally, the permeable cryoprotectants have small molecules with cytotoxicity. In thawing applications, the function of the non-permeable cryoprotectants is ensuring that the osmotic pressure difference between the inner of the cell and the outer of the cell is not too large. In other words, in the thawing application, the inner of the cells is filled with the permeable cryoprotectant when the cells are taken out from the liquid nitrogen, it leads that the osmotic pressure inside the cells is quite high. If these cells are placed into a media directly during the thawing process, the water molecules will enter into the cells at a very fast speed, which causes that the cells would be killed under the osmotic pressure difference. However, if the cells are displaced within a solution containing a non-permeable cryoprotectant during the thawing process, the smaller osmotic pressure differences are capable of reducing the speed of water molecules entering the cells and are capable of making the permeable cryoprotectant within the cell diffuse out of the cell, thus the cryoprotectant could be removed slowly.

Wherein, the types of the chemical include: a second dilution solution and a washing solution. The second dilution solution contains a basic solution and a non-permeable cryoprotectant having a concentration of the second dilution solution lower than the concentration of the first dilution solution, and the washing solution only includes basic solution. Specifically, two sections are capable of being sequentially arranged on the chip, and the second dilution solution and the washing solution are encapsulated in the sections sequentially, wherein the coverage area of each section is larger than the opening area of the recess of the second carrier.

In the embodiment according to the present application, the chemical could be in solid made of hydrogel or in solution encapsulated within the chip. As for the chemical in solid, the second dilution solution and the washing solution in hydrogel, that is, the second dilution hydrogel (DS2 gel) and the washing hydrogel (WS gel) are embedded in the chip. As for the chemical in solution, the dilution solution and the washing solution, in solution form, are encapsulated within the chip by a permeable membrane. In practice, a perforated membrane, mesh or dialysis membrane with appropriate thickness and appropriate pore size could be selected to be arranged on the lower surface of the chip and the solutions could be applied into the section directly, for supporting the solution by perforated membrane, mesh, dialysis membrane, or water-soluble membrane, in order to immobilize the solution in the chip.

In step S212, moving the chip relative to the second carrier, the chemical contacts the recess of the second carrier in a full-covering manner, for removing the cryoprotectant in the recess.

In the case that the chemical in solid hydrogel is immobilized in the chip, the second dilution hydrogel and the washing hydrogel embedded in the chip are moved to the upper side of the recesses in the second carrier in sequence and contact with the biological material to be thawed in the recesses in a full-covering manner, when the chip moves relative to the carrier. Since chemical molecules are capable of diffusing within the hydrogel freely, the embryos and cryoprotectant in the recess are capable of diffusing into the hydrogel when the chemical molecules contact with the recess on the carrier, in order to realize the function of removing the cryoprotectant. In the case of using the second dilution hydrogel, the water is capable re-diffusing into the embryos slowly, in order to remove the non-permeable cryoprotectant molecules around the embryos in sequence. In the case of using the washing hydrogel, the more water is capable of re-diffusing into the embryos slowly, for removing the non-permeable cryoprotectant molecules around the embryos completely and for bring the embryos back to the environment having a normal osmotic pressure. The embryos treated by hydrogel do not contain any cryoprotectants, so as to bring the embryos back to the environment having a normal osmotic pressure, thereby the embryos could be transferred from the carrier to the culture dish and cultured in the incubator.

In the case that the chemical in solution is immobilized within the chip, the embryos are prevented from drifting out by the permeable membrane covering the recess, thus, once the solutions on the two sides of the permeable membrane contact with each other, the diffusion-exchange between the solutions on two sides could be implemented by the penetration of the solution, please refer to the embodiment related to the hydrogel for details.

In brief, no matter in solid or in liquid, when the cryoprotectant is diffused and removed, the chemical is capable of keeping the embryos in the recess, meanwhile, the diffusion-exchange could be implemented and the chemical is prevented from drifting out of the chip. When the entire removal process is completed, the embryos are left in the recess of the carrier, thus the positions of the embryos are capable of being quickly determined and the embryos are capable of being captured, meanwhile, the embryos are capable of being transferred from the carrier to the culture dish and are capable of being cultured in the incubator for culturing. Since the medium composition of the washing hydrogel (or the medium composition of the washing solution) is not necessarily the same as the user's medium composition, the user could put the embryos transferred from the carrier into the user's medium for the final washing firstly as needed, and then these embryos are transferred to the user's culture system for culturing.

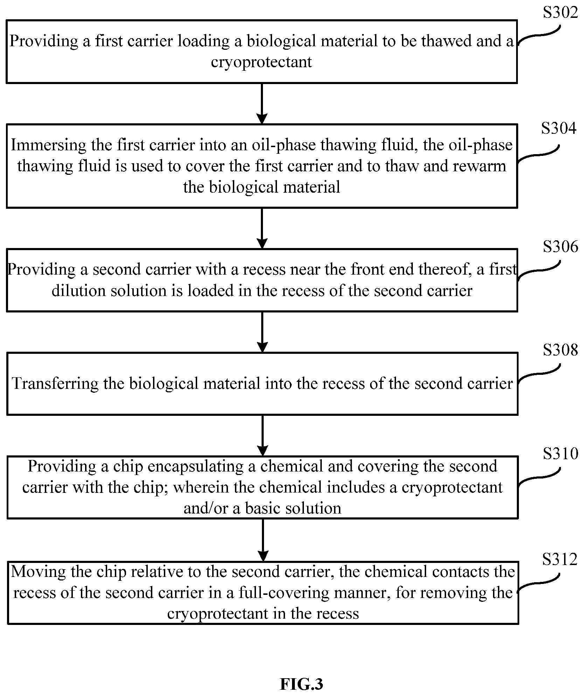

When the thawing process is performed by using an oil-phase thawing fluid, if the embryos are carried by a non-standard carrier, there is a need for further transferring to a standard carrier after the thawing-rewarming process is completed, only by this way, the carrier is capable of cooperating with the chip for removing the cryoprotectant. Refer to FIG. 3, according to one embodiment of the present application, a method of thawing biological materials is also proposed, specifically, this method includes the following steps:
Step S302, providing a first carrier loading a biological material to be thawed and a cryoprotectant;
Step S304, immersing the first carrier in an oil-phase thawing fluid, the oil-phase thawing fluid is used to cover the first carrier and to thaw and rewarm the biological material;

In step S306, providing a second carrier with a recess near the front end thereof, a first dilution solution is loaded in the recess of the second carrier.

The second carrier is capable of matching and cooperating with the chip.

Step S308, transferring the biological material into the recess of the second carrier.

Step S310, providing a chip encapsulating a chemical and covering the second carrier with the chip; wherein the chemical includes a cryoprotectant and/or a basic solution.

Wherein, the chemical may contain a cryoprotectant and/or a basic solution, or the chemical may contain a non-permeable cryoprotectant and/or a basic solution. Preferably, the chemical containing non-permeable cryoprotectants and/or basic solution components could be adopted. And wherein, the chemical covers a partial area of the carrier and does not cover the recess of the carrier, and there is an interstice distance between the chemical and the recess of the carrier.

Step S312, moving the chip relative to the carrier, the chemical contacts the recess of the second carrier in a full-covering manner, for removing the cryoprotectant in the recess.

For the explanation of each term in this embodiment could be made to the same or similar parts of the previous embodiment, and will not be repeated here.

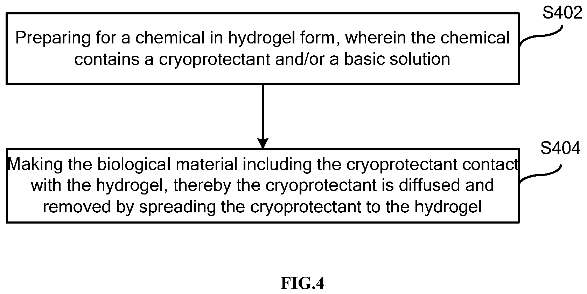

Refer to Fig.4, according to one embodiment of the present disclosure, a method of thawing the biological materials includes the following steps:
Step S402, preparing the chemical in a hydrogel form, wherein the chemical contains a (first) cryoprotectant and/or a basic solution;

Wherein, the chemical may contain a permeable cryoprotectant and/or a basic solution, or the chemical may contain a non-permeable cryoprotectant and/or a basic solution. Preferably, the chemical containing non-permeable cryoprotectants and/or basic solution components could be adopted.

In step S404, making the biological material including the (second) cryoprotectant contact with the hydrogel, thereby the (second) cryoprotectant is diffused and removed by spreading the (second) cryoprotectant to the hydrogel.

Wherein, the chemical includes one or more of thawing solution, dilution solution or washing solution.

Wherein, the preparation of the hydrogel could be implemented by the preparation method based on physical hydrogel, e.g., the preparation method based on sodium alginate hydrogel, gelatin hydrogel or agarose gel, or could be implemented by the preparation method based on chemical hydrogel, e.g., the preparation method based on PEGDA hydrogel or GelMA hydrogel. Moreover, this embodiment relates to preparing a hydrogel based on the thawing solution directly, for removing the cryoprotectant (such as the vitrification solutions) from the embryo at once. However, in the use, according to specific conditions, e.g., the concentration of the thawing solution which is used, it is perfectly safe to divide the thawing solution into multiple hydrogels with the thawing solutions in different concentrations and to use the multiple hydrogels in different concentrations for removing the cryoprotectant in a sequence controllable way, for completing the entire cryoprotectant removal operation. The preparation methods of various forms of hydrogels are described in detail below.

Moreover, the method for preparing agarose gel includes:
Dissolving an agarose in a chemical solution at 80-90°C for preparing a 0.1-6% agarose solution; and obtaining an agarose gel containing chemical by stirring, mixing, cooling, and solidifying.

Moreover, the method for preparing sodium alginate hydrogel includes:
Dissolving sodium alginate in the thawing solution for preparing a 0.3-10% sodium alginate solution; dissolving calcium chloride in the thawing solution for preparing a 0.01M-0.2M calcium chloride solution; providing the calcium chloride solution onto the sodium alginate solution in the chip slowly; solidifying the whole piece of sodium alginate solution into calcium alginate hydrogel, till the calcium chloride has diffused to the bottom of the chip.

Moreover, the method for preparing gelatin hydrogel includes:
Dissolving the gelatin in the thawing solution at 36-45°C for preparing a 1-15% gelatin solution; applying the gelatin solution at 36-50°C into the chip; cooling and solidifying the gelatin solution for forming hydrogel encapsulating thawing solution.

Moreover, the method for preparing GelMA hydrogel includes:
Dissolving GelMA in the thawing solution at 36-50°C for preparing a 1-15% GelMA solution; dissolving a photo initiator in GelMA solution at 36-50°C with a concentration of 0.001-2%; adding the GelMA solution at 36-50°C to the chip; irradiating the GelMA solution at 36-50°C for 2-30 minutes so as to solidify the GelMA solution into a hydrogel.

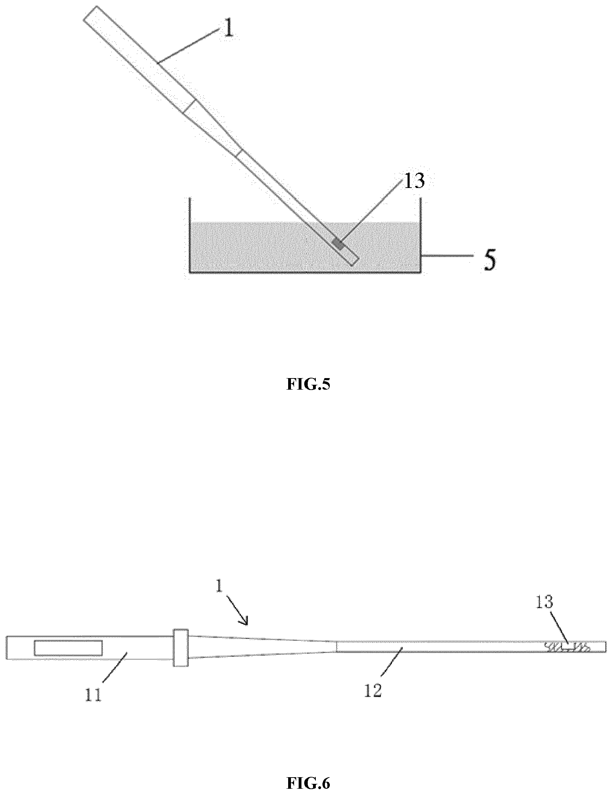

Refer to Figs.5-11, according to an embodiment of the present disclosure, a system used for thawing biological materials includes a carrier 1, a container 5 and a chip 2.

Refer to Fig.6, the carrier 1 is provided with a recess 13 near the front end thereof, the recess 13 contains the biological material to be thawed and a cryoprotectant (VS) for protecting the biological material to be thawed, wherein the biological material could be cells, biological tissues, eggs or embryos, and the cryoprotectant could be vitrification solution. The carrier 1 has a strip-shaped structure including a handle 11, a sheet 12 and a recess 13. Wherein, the recess 13 is near the front end of the sheet 12, and the recess 13 contains the biological material to be thawed and a cryoprotectant (VS) that protects the biological material to be thawed, wherein the biological material could be cells, biological tissues, eggs or embryos, and the cryoprotectant could be vitrification solution. Meanwhile, the size of the recess 13 can be adjusted according to the number of the embryos to be processed, size of the embryos to be processed and the amount of solution received.

In the embodiments of the present application, the carrier 1 may have a strip structure for facilitating the cooperation with the system to perform the thawing-rewarming operation of the embryo and for improving the compatibility of the carrier. At the same time, the handle part of the carrier is preferably designed as a structure has a sufficient width for facilitating to set labels, so as to mark some relevant information of the embryos to be processed, meanwhile, the sheet is made of a plastic material having uniform thickness, transparent material, biological compatibility and good thermal conductivity for ensuring the suitability, so as to hold embryos and the heat transfer speed during subsequent thawing. Similarly, in other embodiments, according to different operating conditions and requirements, the carrier can also be designed in other structural forms, e.g., a flat-plate structure, with recesses.

The container 5 is a vessel containing an oil-phase thawing fluid into it, the form and the structure of the container 5 is not restricted, which can be any vessel with the function of containing the oil-phase thawing fluid, preferably a container. The oil-phase thawing fluid does not contain any non-permeable cryoprotectant. The carrier is immersed in the oil-phase thawing fluid, for immersing the recess of the carrier below the liquid surface, the oil-phase thawing fluid is used to thaw and rewarm the embryos, for rewarming the embryos to a room temperature or 37°C. Since the cryoprotectant and the oil-phase thawing fluid cannot mix together and the density of the oil-phase thawing fluid is lower than the density of the cryoprotectant, the cryoprotectant and the embryo could always be left in the recess, that is, the cryoprotectant in the recess and the embryo to be thawed could always be left in the recess and cannot drift out of the carrier during the thawing-rewarming process.

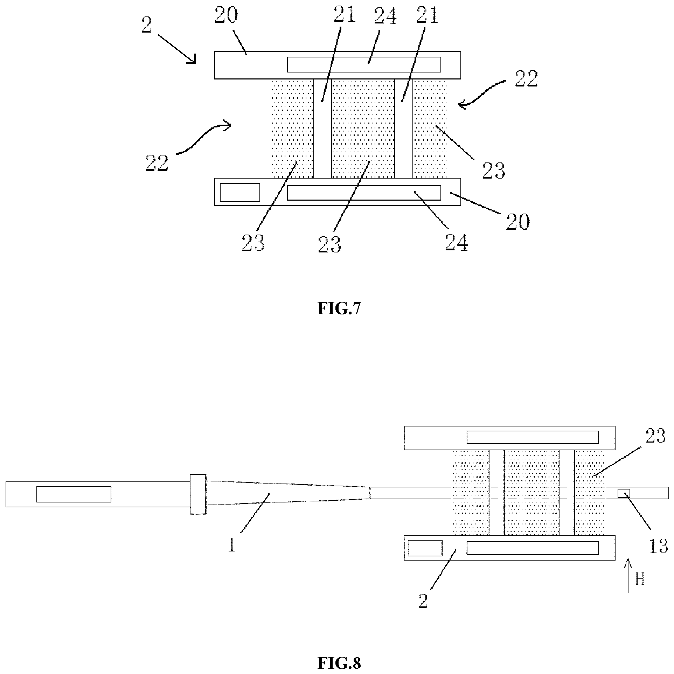

Referring to FIG. 7, the chip 2 can also be called as a biochip, the chip 2 is formed as a plate-frame structure, and a plurality of sections 22 are arranged on the chip 2 in sequence for immobilizing or encapsulating chemicals respectively. In this embodiment, the chip 2 is formed as a frame structure composed of two support plates 20 and two separators 21. Wherein, the two support plates 20 maintain parallel to each other, the two separators 21 maintain parallel to each other and are positioned between the two support plates 20, thereby the section between the two support plates 20 is divided into three sections 22 separated from each other used to arrange the different chemicals to be delivered respectively.

Optionally, the connection between the separators 21 and the support plate 20 in the chip 2 could be designed as an active connection, thereby the size of the section 22 could be freely adjusted. For example, by providing sliding recesses on the opposite surfaces of the two support plates 20, the ends of the separators 21 are capable of being inserted into the sliding recesses, thus the position of the separators 21 can be freely adjusted in the sliding recess, thereby the size of the section is adjustable, for carrying the chemical to be delivered in different dosages.

The type of chemical on the chip include: thawing solution, dilution solution and washing solution. Wherein, the chemical encapsulated on the chip could be in solid or in liquid. As for the chemical in solid, the chemical is made into hydrogels, that is, the thawing hydrogels, the dilution hydrogels and the washing hydrogels embedded into the chip. As for the chemical in solution, the thawing solution, the dilution solution and the washing solution, in solution, are encapsulated within the chip by a permeable membrane. In practice, a perforated membrane, mesh or dialysis membrane with suitable thickness and suitable pore size could be chosen to be provided on the lower surface of the chip, meanwhile, the solutions could be supplied into the sections directly, thereby, the solutions are capable of being supported by the perforated membrane, the mesh, the dialysis membrane, or the water-soluble membrane, and thus the solutions are capable of being immobilized in the chip.

Refer to Fig.8, the carrier 1 is covered by the chip 2 encapsulating chemical, at this time, the chemical merely covers some parts of the carrier and does not cover the recesses of the carrier, and there is an interstice distance between the chemical and the recesses of the carrier. The chip is capable of being moved relative to the carrier, thus the thawing solution, the dilution solution and the washing solution in solid or liquid are capable of sequentially moving to the top of the recess of the carrier and are capable of contacting to the biological material to be thawed in the recess in a full-covering manner, by this way, the cryoprotectant in the recess could be removed. During the removal process, the embryos are blocked in the recess, meanwhile the diffusion exchange with the chemical in the recess is completed, and the chemical cannot drift out of the chip. When the entire removal process is completed, there is not any cryoprotectant and a normal osmotic pressure environment is recovered in a sequential manner, and at this time, the embryos are capable of being left in the recess of the carrier, the positions of the embryos are capable of being quickly determined and the embryos are capable of being captured, thereby the embryos could be transferred from the carrier to the culture dish and cultured in the incubator for culturing.

In this embodiment, three sections are provided on the chip for respectively placing solutions having cryoprotectants with different concentrations, thus the concentration relationship among the different solutions to be delivered to the embryos in sequence could be controlled accurately, for ensuring the accuracy of the solution delivery. In some other embodiments, according to the requirements for the number of types and the concentration controlling of the chemicals to be delivered, the number of sections on the chip can be adjusted accordingly as per the specific demand, for controlling the concentration gradients between the chemicals in adjacent sections precisely, so as to guarantee the delivery accuracy of the chemicals.

It should be noted that, although only one recess is provided on the carrier according to this embodiment, the carrier absolutely could be provided with a plurality of recesses based on the number of the embryos to be processed, by this way, a plurality of embryos are capable of being processed simultaneously, for improving the processing efficiency.

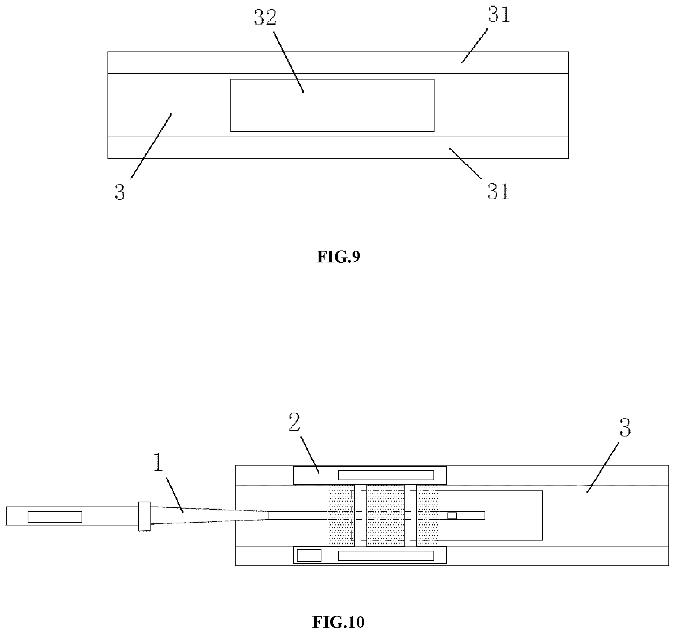

Refer to Figs.9 and 10, the biological material thawing system may further include a substrate 3 having a U-shape structure, wherein the middle area of the substrate 3 is used to support and fix the carrier 1, the two side walls along the length direction of the substrate are capable of being used as guiding rails 31, for supporting and adsorb-fixing the support plate 20 of the chip 2, meanwhile, the positional relationship between the chip 2 and the upper surface of the carrier 1 could be adjusted by changing the heights of the guiding rails 31, for ensuring an efficient contact between the hydrogel and the solution in the recess.

In other embodiments, the two guiding rails of the substrate 3 are capable of being provided with one guiding step respectively. Thereby, the guiding steps are capable of being used to guide the chip during the movement of the chip when the chip is arranged between two guiding steps, for improving the directional accuracy of chip on the substrate.

Between the rails 31 and the chip 2, a detachable fixed connection in the form of magnetic attraction is provided. For example, the rails 31 are capable of being made of ferromagnetic metal and a magnet 24 is capable of being provided at a corresponding position on the support plate 20, so as to form a magnetically attached fixed connection between the rails 31 and the chip 2. More preferably, the rails could be provided in an electromagnet structure, by this way, the connection and disconnection between the substrate and the chip could be realized through electrical controlling, thus the convenience of operation could be improved.

By providing a substrate, the carrier is capable of being supported and fixed, e.g., by the stick connection or snap connection between the substrate and the carrier, for ensuring the stability of the position of the carrier during the entire operation; furthermore, the chip is also capable of being supported and fixed for maintaining an effective contact between the chip and the carrier, so as to avoid the accidental separation which affects the normal operation during the relative movement of the chip and the carrier. At the same time, the substrate is also capable of collecting the solution drifting outside of the recess, so as to prevent the pollution in the surrounding environment caused by the solution drifting outside.

Refer to FIG.9, the middle area of the substrate 3 is further provided with a light-transmitting area 32, that is, the light-transmitting area is formed by applying light-transmitting materials to an area corresponding to the recess 13 in the substrate 3. At this time, with the help of the light penetrated through the light-transmitting area, the substrate could bring the carrier and the chip under the microscope, thus the carrier and the chip are capable of being observed directly, for observing the chemical delivery process in real time, so as to control the chemical delivery progress accurately.

Moreover, according to actual operation requirements, the light-transmitting area could be formed of conventional light-transmitting materials to merely meet the light-transmitting requirements, at the same time, the light-transmitting area could be formed of light-transmitting materials with heating functions, e.g., heating glass materials, so as to meet the requirements for light-transmitting and the requirement for temperature-controlling simultaneously.

Furthermore, although only one carrier and one chip are provided on the substrate according to this embodiment, a plurality of carriers are capable of being arranged on the substrate in a side-by-side manner simultaneously according to the number of embryos to be processed and the section width of the chip, i.e., the coverage width of the hydrogel, thus the chemical delivery operations of the embryos on the plurality of carriers could be completed simultaneously, thereby the operation efficiency could be improved.

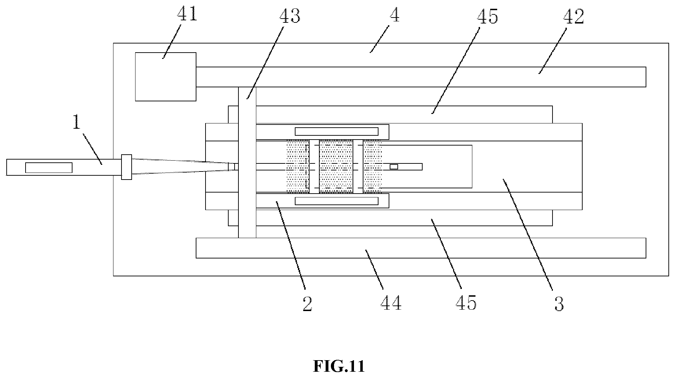

Refer to Fig. 11, the biological material thawing system may further include a base 4 used for directly supporting and fixing the substrate 3, wherein the base 4 is provided with a driving unit composed of a stepping motor 41, a screw level 42 and a push rod 43. Wherein, the substrate 3 is arranged on the base 4 and is arranged in parallel with the screw level 42, the push rod 43 is sleeved on the screw level 42 and is capable reciprocating along the screw level 42 under the driving of the stepping motor 41, meanwhile, the push rod 43 is fixed to the chip 2.

At this time, the stepping motor 41 is capable of driving the pushing rod 43 to move along the screw level 42 horizontally in a reciprocate manner, thus the chip 2 is capable of reciprocating with respect to the carrier 1 horizontally, by this way, the hydrogel in different sections on the chip 2 is capable of connecting with the solutions in the recess 13 in sequence, for realizing an automatic controlling. Moreover, by controlling the movement of the stepping motor 41, the contacting time of different hydrogels in the chip 2 and the solution in the recess 13 is capable of being controlled precisely, so as to control the delivering time of solutions in hydrogel and the connecting time of different solutions and the embryos precisely for further improving the accuracy of the delivery of different solutions to the embryos.

Moreover, in other embodiments, the screw level and the push rod could be replaced by a rail slider structure to form the driving unit, and the reciprocating movement of the slider along the rails is capable of enabling the chip move relative to the carrier.

Continuing to refer to Fig. 11, the base 4 is further provided with a plain shaft 44, the plain shaft is a smooth and cylindrical shaft. The plain shaft 44 remains parallel with the screw level 42 and is connected to the free end of the push rod 43, so as to provide an auxiliary guidance for the reciprocating movement of the push rod 43, thereby the stability of the movement of the chip 2 driven by the push rod 43 could be improved and the stability of the contacting process between the hydrogel and the solution in the recess could be improved.

As shown in the figure, a hollow area 45 is further provided in the middle of the base 4, that is, the area corresponding to the light-transmitting area in the chip 3 has a hollow structure. By this way, it can be ensured that the light passes through the base 4 smoothly and projects onto the light-transmitting area of the chip 3, for ensuring the normal use of a microscope. Similarly, according to the actual use in different environments, the hollow area could be made of light-transmitting materials, e.g., light-transmitting glass, to implement the light-transmission, furthermore, the hollow area could also be made of light-transmitting materials with heating functions, such as heating glass, to implement the light transmission and temperature control simultaneously.

Furthermore, although the foregoing embodiments describing the present disclosure take the operation of delivering different chemical solutions in the process of thawing embryos for examples, a person skilled in the art should appreciate that the chemical delivery system according to the present disclosure could be used to deliver the chemicals into other biological materials or basic solutions accurately, for example, as the powdered chemicals are delivered into the solution, the amount of the single-delivery chemical could be temporarily supported by the water-soluble membrane; meanwhile, during the movement of the chip relative to the carrier, once the water-soluble membrane comes into contact with the solution and dissolves, a quantitative amount of chemicals is capable of being directly released into the solution in time, thereby a precise delivery of the chemicals could be completed.

According to an embodiment of the present application, a system of thawing biological materials is further provided. The system includes: a first carrier, a container, a second carrier, a capillary tube, and a chip. Specifically:
The first carrier contains the biological material to be thawed and the cryoprotectant. In the present disclosure, the shape and the resulting of the first carrier are not limited, that is, the first carrier may have a recess and the recess is used to carry the biological material; alternatively, the first carrier could be not provided with a recess, the biological material could be carried in other manners.

The container contains the thawing solution, the thawing solution is used to cover the first carrier immersed therein and to thaw-rewarm the biological material, thereby the biological material is capable of being separated from the first carrier;
A recess is provided near the front end of the second carrier, and the first dilution solution is contained in the recess of the second carrier;
The capillary tube is used to transfer the biological material into the recess of the second carrier;
The chip encapsulates chemicals, the chemicals include cryoprotectants and/or basic solution; the chip is capable of moving relative to the second carrier, thereby the chemicals are capable contacting the recesses of the second carrier, in a full-covering manner, for removing the cryoprotectant in the recess.

According to the embodiments of the present application, the concentration of the cryoprotectant contained in the chemical is less than the concentration of the cryoprotectant in the recess. In practice, the cryoprotectant contained in the chemicals could be a non-permeable cryoprotectant or a permeable cryoprotectant. Specifically, when the cryoprotectant in embryos is removed by the chemical, the chemical may contain cryoprotectants and/or basic solution, or the chemical may contain non-permeable cryoprotectants and/or basic solution. In order to obtain a better removal effect, the chemical containing non-permeable cryoprotectants and/or basic solution could be used.

Wherein, the type of the chemical include: a second dilution solution and a washing solution, the second dilution solution contains a basic solution and a non-permeable cryoprotectant with a concentration lower than the concentration of the first dilution solution, and the washing solution only contains the basic solution. The chemical could be in solid or solution encapsulated in the chip. As for the chemical in solid, the second dilution solution and the washing solution are immobilized in hydrogel, in other words, the second dilution hydrogel and the washing hydrogel are embedded in the chip. When the chip moves relative to the carrier, the second dilution hydrogel and the washing hydrogel are capable of moving to the top of the recesses in sequence and are capable of sequentially contacting the recesses in a full-covering manner. As for the chemical in solution, the dilution solution and the washing solution in solution are encapsulated within the chip by a permeable membrane. In practice, a perforated membrane, mesh or dialysis membrane with appropriate thickness and appropriate pore size can be chosen so as to be arranged on the lower surface of the chip and to directly apply the solution into the section, for supporting the solution by perforated membrane, mesh, dialysis membrane, or water-soluble membrane, in order to immobilize the solutions in the chip.

With regards to the shape and structure, the second carrier and the chip in this embodiment are similar to the second carrier and the chip in the previous embodiments, and the further description will not be repeated here. In this embodiment, the embryos are immersed in the non-oil phase thawing fluid by the first carrier for performing the thawing-rewarming process, as the first carrier and the chip (and other components in the system) cannot be compatible with each other, the embryos are needed to be transferred to the second carrier which is compatible with the chip (and the other components in the system), for removing the cryoprotectant.

It should be noted that, the system according to this embodiment may also include components such as a substrate and a base, and the further description will not be repeated here.

According to the present disclosure, the operation steps in the method and the structural features in the system are corresponding and cross-referenced, thus the further descriptions will not be repeated here.

According to the present disclosure, the beneficial effects could be obtained include:
(1) in comparison with the existing manual thawing processes, the present disclosure proposes a standardized process driven by a machine, wherein the removal rate of the cryoprotectant is adjustable. The workflow of the thawing process is stable each time; and the process is simple, in conjunction with the freezing method previously proposed by the applicant, an embryologist only needs to transfer the embryos from the carrier to a culture after the thawing process is completed by the machine, rather than applies the embryos to the carrier; the embryos are provided on a fixed position on the carrier through the whole thawing-rewarming process, thereby, the embryologist does not need to look for the embryos in the liquid with large volume and high throughput.
(2) in comparison with the existing microfluidics, there is not any dead volume in the carrier according to the present disclosure, meanwhile, the position of the embryo on the carrier allows embryologists to directly recovery the embryos with glass capillaries.
(3) in comparison with the existing robot (which is capable of simulating human's hand), according to the present disclosure, a high throughput is provided and the core function only needs a linear moving motor platform having lower cost and does not need machine vision to distinguish the embryos.

Those skilled in the art should understand that, the embodiments of the present disclosure could be implemented as a method, a system, or a computer program product. Therefore, the present disclosure can be completely implemented in hardware, software, or a combination thereof. Moreover, the present disclosure can be implemented in computer program product which could be provided on one or more computer-readable storage media (including, but not limited to, magnetic disk memory, CD-ROM, optical memory and so on) having computer-readable codes.

The above descriptions merely provide some embodiments according to the present disclosure and are not used to limit the present disclosure. For those skilled in the art, the present disclosure could be implemented by various modifications and changes. Any modification, equivalent replacement, improvement, etc. made within the spirit and principle of the present disclosure shall be included in the scope of the claims of the present disclosure.

## Claims

1. A system of thawing biological materials, comprising:
a carrier with a recess located near a front end of the carrier, in which a biological material to be thawed and a cryoprotectant are loaded;
a container, for loading an oil-phase thawing fluid used to cover the recess of the carrier immersed therein and to thaw and rewarm the biological material;
a chip encapsulating a chemical including a cryoprotectant and/or a basic solution; with the chip moving relative to the carrier, the chemical contacts the recess in a full-covering manner for removing the cryoprotectant in the recess.

2. The system according to claim 1, wherein the cryoprotectant contained in the chemical is a non-permeable cryoprotectant.

3. The system according to claim 2, wherein the chemical includes a thawing solution, a dilution solution and a washing solution, which are made in form of hydrogels; the chip is provided with a plurality of sections used for embedding a thawing solution, a dilution solution and a washing solution in sequence, wherein coverage area of each section is larger than opening area of the recess; while the chip moves relative to the carrier, the thawing solution, the dilution solution and the washing solution move to upper side of the recess sequentially and contact the recess in a full-covering manner sequentially.

4. The system according to claim 2, wherein the chemical includes a thawing solution, a dilution solution and a washing solution, which are made in form of solutions; the chip is provided with a permeable membrane for supporting the thawing solution, the dilution solution and the washing solution; wherein the permeable membrane is one of a perforated membrane, a mesh, a dialysis membrane or a water-soluble membrane.

5. The system according to claim 3 or 4, wherein prior to the chip moving relative to the carrier, the chemical covers a part area of the carrier excluding the recess of the carrier, and an interstice distance is provided between the chemical and the recess of the carrier.

6. The system according to claim 3 or 4, further comprising a substrate, a middle area of the substrate is used to place the carrier, and two rails paralleling with each other for supporting and fixing the chip are provided along a length direction of the substrate, to keep contacting between a lower surface of the chip and an upper surface of the carrier.

7. A system of thawing biological materials, wherein, comprising:
a first carrier loading a biological material to be thawed and a cryoprotectant;
a container loading a thawing solution used to cover the first carrier immersed therein and to thaw and rewarm the biological material to separate the biological material from the first carrier;
a second carrier with a recess located near a front end of the second carrier, in which a first dilution solution is loaded;
a capillary tube used to transfer the biological material into the recess of the second carrier;
a chip encapsulating a chemical including a cryoprotectant and/or a basic solution; with the chip moving relative to the second carrier, the chemical contacts the recess of the second carrier in a full-covering manner for removing the cryoprotectant in the recess.

8. The system according to claim 1, wherein the cryoprotectant contained in the chemical is a non-permeable cryoprotectant.

9. The system according to claim 8, wherein the chemical includes a second dilution solution and washing solution made in form of hydrogels, the second dilution solution contains a non-permeable cryoprotectant with a concentration lower than the concentration of the non-permeable cryoprotectant in the first dilution solution; the chip is provided with a plurality of sections for embedding a second dilution hydrogel and a washing hydrogel in sequence, wherein coverage area of each section is larger than opening area of the recess; while the chip moves relative to the carrier, the second dilution hydrogel and the washing hydrogel move sequentially to an upper side of the recess and contact the recess in a full-covering manner sequentially.

10. The system according to claim 8, wherein the chemical includes a second dilution solution and a washing solution, which are made in form of solutions; wherein a concentration of the non-permeable cryoprotectant in the second dilution solution is lower than a concentration of the non-permeable cryoprotectant in the first dilution solution; the chip is provided with a permeable membrane, and the permeable membrane is used to support the second dilution solution and the washing solution in solution form; wherein the permeable membrane is one of a perforated membrane, a mesh, a dialysis membrane or a water-soluble membrane.

11. The system of claim 9 or 10, wherein prior to the chip moving relative to the second carrier, the chemical covers a part area of the second carrier excluding the recess of the second carrier, and an interstice distance is provided between the chemical and the recess of the second carrier.

12. The system of claim 9 or 10, further comprising a substrate, a middle area of the substrate is used to place the second carrier, and two rails paralleling with each other for supporting and fixing the chip are provided along the length direction of the substrate, to keep contacting between a lower surface of the chip and an upper surface of the second carrier.

13. A method of thawing biological materials, comprising:
providing a carrier with a recess near the front end thereof, in which a biological material to be thawed and a cryoprotectant are loaded;
immersing the recess of the carrier in an oil-phase thawing fluid, the oil-phase thawing fluid is used to cover the recess of the carrier and to thaw and rewarm the biological material;
providing a chip encapsulating a chemical including a cryoprotectant and/or a basic solution;
removing the carrier from the oil-phase thawing fluid, and covering the carrier with the chip;
moving the chip relative to the carrier, the chemical contacts the recess in a full-covering manner for removing the cryoprotectant in the recess.

14. The method according to claim 13, wherein the cryoprotectant contained in the chemical is a non-permeable cryoprotectant.

15. The method according to claim 14, wherein prior to the chip moving relative to the carrier, the chemical covers a part area of the carrier excluding the recess of the carrier, and an interstice distance is provided between the chemical and the recess of the carrier.

16. The method according to claim 14, wherein the chemical includes a thawing solution, a dilution solution and a washing solution made in form of hydrogels; the chip is provided with a plurality of sections for embedding a thawing hydrogel, a dilution hydrogel and a washing hydrogel in sequence, wherein coverage area of each section is larger than opening area of the recess; while the chip moves relative to the carrier, the thawing solution, the dilution solution and the washing solution move sequentially to an upper side of the recess and contact the recess in a full-covering manner sequentially.

17. The method according to claim 14, wherein the chemical includes a thawing solution, a dilution solution, and a washing solution made in form of solutions; the chip is provided with a permeable membrane for supporting the thawing solution, the dilution solution and the washing solution; wherein the permeable membrane is one of a perforated membrane, a mesh, a dialysis membrane or a water-soluble membrane.

18. A method of thawing biological materials, comprising:
providing a first carrier loading a biological material to be thawed and a cryoprotectant;
immersing the first carrier into a thawing solution, the thawing solution is used to cover the first carrier and to thaw and rewarm the biological material;
providing a second carrier with a recess near the front end thereof, a first dilution solution is loaded in the recess of the second carrier;
transferring the biological material into the recess of the second carrier;
providing a chip encapsulating a chemical and covering the second carrier with the chip; wherein the chemical includes a cryoprotectant and/or a basic solution;
moving the chip relative to the second carrier, the chemical contacts the recess of the second carrier in a full-covering manner, for removing the cryoprotectant in the recess.

19. The method according to claim 18, wherein the cryoprotectant contained in the chemical is a non-permeable cryoprotectant.

20. The method according to claim 19, wherein prior to the chip moving relative to the carrier, the chemical covers a part area of the second carrier excluding the recess of the second carrier, and an interstice distance is provided between the chemical and the recess of the second carrier.

21. The method according to claim 19, wherein the chemical includes a second dilution solution and a washing solution made in form of hydrogels, the second dilution solution contains a non-permeable cryoprotectant with a concentration lower than a concentration of the non-permeable cryoprotectant contained in the first dilution solution; the chip are provided a plurality of sections for embedding a second dilution hydrogel and a washing hydrogel in sequence, wherein coverage area of each section is larger than opening area of the recess; while the chip moves relative to the carrier, the second dilution solution and the washing solution move sequentially to an upper side of the recess and contact the recess in a full-covering manner sequentially.

22. The method according to claim 19, wherein the chemical includes a second dilution solution and a washing solution made in form of solutions; wherein the concentration of the non-permeable cryoprotectant in the second dilution solution is lower than the concentration of the non-permeable cryoprotectant in the first dilution solution; the chip is provided with a permeable membrane to support the second dilution solution and the washing solution; wherein the permeable membrane is one of a perforated membrane, a mesh, a dialysis membrane or a water-soluble membrane.

23. A method of thawing biological materials, comprising:
preparing for a chemical in hydrogel form, wherein the chemical contains a cryoprotectant and/or a basic solution;
making the biological material including the cryoprotectant contact with the hydrogel, thereby the cryoprotectant is diffused and removed by spreading the cryoprotectant to the hydrogel.

24. The method according to claim 23, wherein the cryoprotectant contained in the chemical is a non-permeable cryoprotectant.

25. The method according to claim 23, wherein the step of preparing for the chemical into a form of hydrogel comprises:
dissolving an agarose into a chemical solution at 80-90°C for preparing for a 0.1-6% agarose solution;
obtaining an agarose gel containing the chemical by stirring, mixing, cooling, and solidifying.

26. The method according to claim 23, wherein the step of preparing the chemical into a hydrogel structure comprises:
dissolving sodium alginate into the chemical solution for preparing for a 0.3-10% sodium alginate solution;
dissolving calcium chloride in the chemical solution for preparing a 0.01-0.2M calcium chloride solution;
providing the calcium chloride solution onto the sodium alginate solution in the chip;
when the calcium chloride is diffused to the bottom of the chip, solidifying the whole piece of sodium alginate solution into calcium alginate hydrogel.

27. The method according to claim 23, wherein the step of preparing for the chemical into a hydrogel structure comprises:
dissolving gelatin into a chemical solution at 36-45°C to make a 1-15% gelatin solution;
applying the gelatin solution at 36-50°C into the chip;
cooling and solidifying the gelatin solution for configuring a hydrogel with the chemical.

28. The method according to claim 23, wherein the step of preparing the chemical into a hydrogel structure comprises:
dissolving GelMA into the chemical solution at 36-50°C for preparing a 1-15% GelMA solution;
dissolving a photo initiator into the GelMA solution at 36-50°C with a concentration of 0.001-2%;
applying the GelMA solution at 36-50°C to the chip;
irradiating the GelMA solution at 36-50°C for 5-30 minutes for solidifying the GelMA solution into a hydrogel.

29. The method according to any one of claims 23 to 28, wherein the chemicals include one or more of a thawing solution, a dilution solution or a washing solution.

30. A method of thawing biological materials, wherein, comprising:
immersing a biological material into an oil-phase thawing fluid for thawing and rewarming.
